(19) Europäisches Patentamt
European Patent Office
Office européen des brevets

(11) Veröffentlichungsnummer: **0 679 696 A1**

(12) # EUROPÄISCHE PATENTANMELDUNG

(21) Anmeldenummer: **95105626.6**

(22) Anmeldetag: **13.04.95**

(51) Int. Cl.[6]: **C09B 62/513**, C09B 62/09, C07D 213/82

(30) Priorität: **25.04.94 DE 4414314**

(43) Veröffentlichungstag der Anmeldung: **02.11.95 Patentblatt 95/44**

(84) Benannte Vertragsstaaten: **CH DE GB LI**

(71) Anmelder: **HOECHST AKTIENGESELLSCHAFT**
**Brüningstrasse 50**
**D-65929 Frankfurt am Main (DE)**

(72) Erfinder: **Reiher, Uwe, Dr.**
**Sachsenring 8**
**D-65719 Hofheim (DE)**
Erfinder: **Russ, Werner Hubert, Dr.**
**Wingertstrasse 8a**
**D-65439 Flörsheim (DE)**

(54) **Wasserlösliche Pyridon-Disazoverbindungen, Verfahren zu ihrer Herstellung und ihre Verwendung als Farbstoffe.**

(57) Es werden neue Disazoverbindungen entsprechend der nachstehend angegebenen und definierten allgemeinen Formel (1) und deren als Vorprodukt dienende Verbindung 1,6-Di-(3-carbamoyl-4-methyl-6-hydroxy-2-on-pyrid-1N-yl)-hexan beschrieben. Die Disazoverbindungen stellen Farbstoffe dar, die faserreaktive Eigenschaften besitzen und auf hydroxy- und/oder carbonamidgruppenhaltigem Fasermaterial, wie beispielsweise Cellulosefasern, Wolle und synthetischen Polyamidfasern, farbstarke grünstichig bis rotstichig gelbe Färbungen liefern.

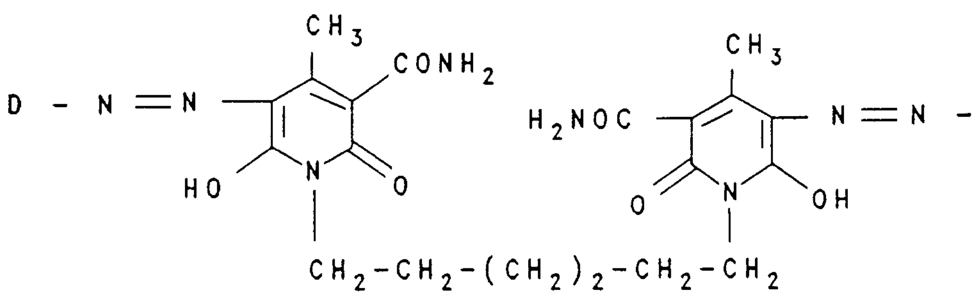

(1)

in welcher D ein durch eine faserreaktive Gruppe der Vinylsulfonreihe substituierter 2-Sulfo-phenyl- oder Sulfo-naphthyl-Rest oder eine Gruppe der Formel

EP 0 679 696 A1

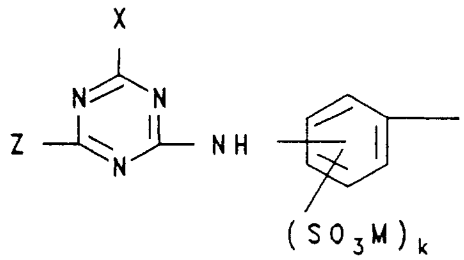

ist, in welcher M Wasserstoff oder ein Alkalimetall ist, k die Zahl Null, 1 oder 2 ist, X Chlor, Fluor, Hydroxy, gegebenenfalls durch Heterogruppen unterbrochenes Alkoxy, Cycloalkoxy, Benzyloxy, Sulfomethoxy, β-Sulfoethoxy, Amino, gegebenenfalls durch Heterogruppen unterbrochenes Alkylamino, Cycloalkylamino, Benzylamino, Sulfomethylamino, β-Sulfoethylamino, Cyanoamino oder durch Sulfo substituiertes Phenyl- oder Naphthylamino ist und Z Chlor, Fluor oder ein durch eine faserreaktive Gruppe der Vinylsulfonreihe substituierter Alkylamino- oder Dialkylamino-Rest oder ein gegebenenfalls durch eine faserreaktive Gruppe der Vinylsulfonreihe substituierter Phenyl- oder Naphthylrest, die auch weitere in faserreaktiven Farbstoffen üblichen Substituenten enthalten können.

Die Erfindung liegt auf dem technischen Gebiet der faserreaktiven Farbstoffe.

Aus der Praxis des Färbens mit faserreaktiven Farbstoffen ergeben sich erhöhte Anforderungen an die Qualität der Färbungen und an die Wirtschaftlichkeit der Färbeprozesse. Infolgedessen bestand weiterhin Bedarf nach neuen faserreaktiven Farbstoffen, die verbesserte Eigenschaften besitzen. Insbesondere für die Herstellung von Färbungen mit gelber Nuance sind faserreaktive Farbstoffe gefragt, die solche Färbungen mit hohen Echtheiten liefern. Aus diesem Grunde hat man bereits in den japanischen Patentanmeldungs-Veröffentlichungen Sho-57-161175 und Sho-58-168660 Pyridon-Disazofarbstoffe zur Herstellung echter Färbungen vorgeschlagen.

Es wurden nunmehr neue Pyridon-Disazoverbindungen gefunden, die der allgemeinen Formel (1)

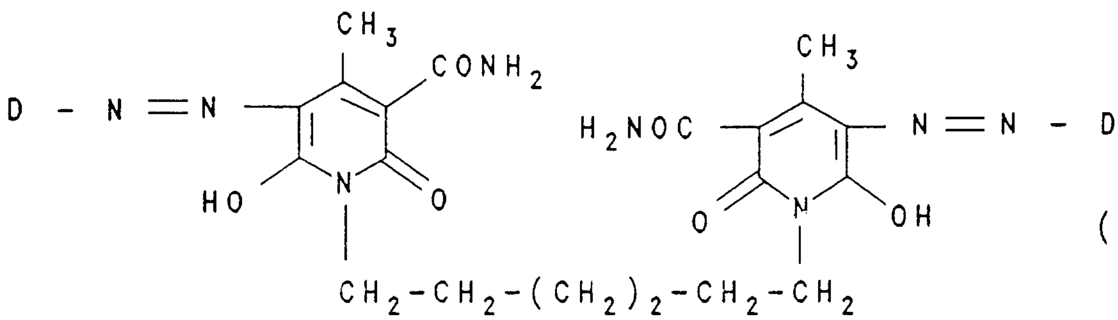

(1)

entsprechen, in welcher bedeuten:

D ist eine Gruppe der allgemeinen Formel (2a), (2b) oder (2c)

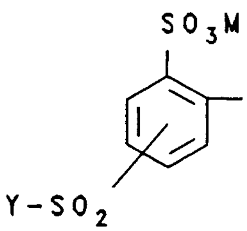

(2a)

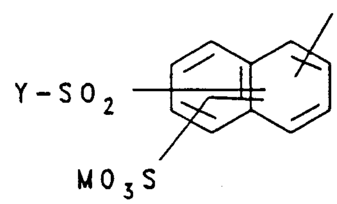

(2b)

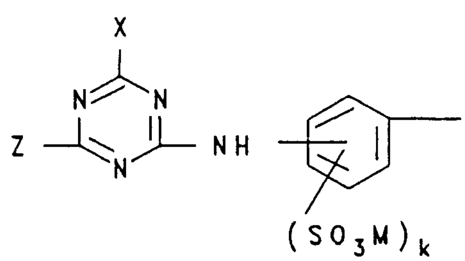

(2c)

in welchen

- Y Vinyl ist oder Ethyl ist, das in $\beta$-Stellung einen Substituenten enthält, der durch Alkali unter Bildung der Vinylgruppe eliminierbar ist,
- M Wasserstoff oder ein Alkalimetall, wie Natrium, Kalium oder Lithium, ist,
- k die Zahl Null, 1 oder 2 ist (im Falle von k gleich Null steht diese Gruppe für ein Wasserstoffatom), wobei im Falle von k gleich Null die nachstehende Gruppe X oder Z bevorzugt eine Sulfogruppe enthält,
- X Chlor, Fluor, Hydroxy, Alkoxy von 1 bis 10 C-Atomen, bevorzugt von 1 bis 4 C-Atomen, wie Propoxy, Ethoxy und Methoxy, oder Cycloalkyloxy von 5 bis 8 C-Atomen ist oder Alkoxy von 3 bis

8 C-Atomen bedeutet, dessen Alkylrest durch eine oder zwei Heterogruppen unterbrochen ist, die aus den Gruppen -O- , -NH- , -CO-NH- und -NH-CO- , hiervon bevorzugt -O- und -NH- , ausgewählt sind, oder Benzyloxy, Sulfomethoxy, $\beta$-Sulfoethoxy, Amino, Alkylamino von 1 bis 10 C-Atomen, bevorzugt von 1 bis 4 C-Atomen, wie Propylamino, Ethylamino und Methylamino, oder Cycloalkylamino von 5 bis 8 C-Atomen, wie Cyclohexylamino, ist oder Alkylamino von 3 bis 8 C-Atomen bedeutet, dessen Alkylrest durch eine oder zwei Heterogruppen unterbrochen ist, die aus den Gruppen -O- , -NH- , -CO-NH- und -NH-CO- , hiervon bevorzugt -O- und -NH- , ausgewählt sind, oder Benzylamino, Sulfomethylamino, $\beta$-Sulfoethylamino oder Cyanoamino ist, hiervon bevorzugt Fluor, Chlor oder Cyanoamino bedeutet, oder Monosulfo-phenylamino, Disulfo-phenylamino, Monosulfo-naphth-2-ylamino, Disulfo-naphth-2-ylamino oder Trisulfo-naphth-2-ylamino ist und

Z Chlor, Fluor oder eine Gruppe der allgemeinen Formel (3a), (3b), (3c) oder (3d)

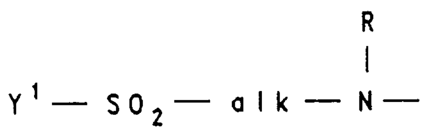
(3a)

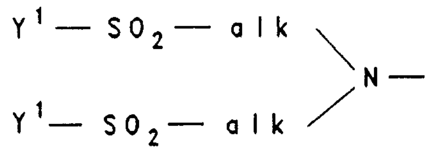
(3b)

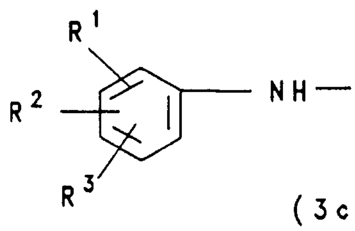
(3c)

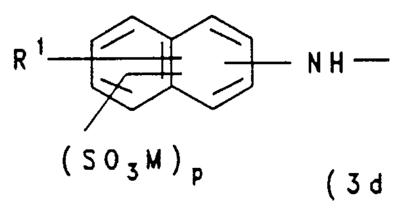
(3d)

ist, in welchen

R Wasserstoff, Phenyl oder durch Sulfo, Ethyl, Ethoxy, Methyl oder Methoxy substituiertes Phenyl ist,

alk Alkylen von 1 bis 8 C-Atomen, bevorzugt von 2 bis 4 C-Atomen, ist, wobei das Alkylen bevorzugt geradkettig ist, oder Alkylen von 3 bis 8 C-Atomen ist, das durch eine oder zwei Heterogruppen unterbrochen ist, die aus den Gruppen -O- , -NH- , -CO-NH- und -NH-CO- , hiervon bevorzugt -O- und -NH- , ausgewählt sind, und ist bevorzugt 1,2-Ethylen oder 1,3-Propylen,

$Y^1$ Vinyl ist oder Ethyl bedeutet, das in $\beta$-Stellung durch einen Substituenten substituiert ist, der durch Alkali unter Bildung der Vinylgruppe eliminierbar ist,

$R^1$ Wasserstoff, Carboxy, Sulfo oder eine Gruppe der allgemeinen Formel $-SO_2-Y^1$ mit $Y^1$ der obengenannten Bedeutung ist,

$R^2$ Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Chlor, Brom, Carboxy, Sulfo oder Nitro und bevorzugt Wasserstoff, Alkyl von 1 bis 4 C-Atomen, Alkoxy von 1 bis 4 C-Atomen oder Sulfo ist,

$R^3$ Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Chlor, Brom, Carboxy oder Sulfo und bevorzugt Wasserstoff, Alkoxy von 1 bis 4 C-Atomen oder Sulfo ist,

M eine der obengenannten Bedeutungen besitzt und

p für die Zahl 1 oder 2 steht,

wobei in Formel (3d) die Gruppe -NH- bevorzugt in $\beta$-Stellung an den Naphthalinkern gebunden ist.

In Formel (2a) ist die Gruppe $Y-SO_2-$ bevorzugt in para-Stellung und insbesondere bevorzugt in meta-Stellung zur Gruppe $-SO_3M$ , in Formel (2b) die zur Azogruppe führende freie Bindung bevorzugt in $\beta$-Stellung an den Naphthalinkern und in Formel (2c) die Triazinylaminogruppe in meta- oder para-Stellung zur an die Azogruppe führende freie Bindung gebunden. Bevorzugt ist einer der Substituenten in dem Rest der obigen Formel (2c) eine Sulfo- oder Carboxygruppe.

In den obengenannten allgemeinen Formeln können die einzelnen Formelglieder im Rahmen ihrer Bedeutung zueinander gleiche oder voneinander verschiedene Bedeutungen haben.

Alkalisch eliminierbare Substituenten, die in β-Stellung der Ethylgruppe von Y und $Y^1$ stehen, sind beispielsweise Halogenatome, wie Brom und Chlor, Estergruppen organischer Carbon- und Sulfonsäuren, wie von Alkylcarbonsäuren, gegebenenfalls substituierter Benzolcarbonsäuren und gegebenenfalls substituierter Benzolsulfonsäuren, wie die Gruppen Alkanoyloxy von 2 bis 5 C-Atomen, hiervon insbesondere Acetyloxy, Benzoyloxy, Sulfobenzoyloxy, Phenylsulfonyloxy und Toluylsulfonyloxy, des weiteren saure Estergruppen anorganischer Säuren, wie der Phosphorsäure, Schwefelsäure und Thioschwefelsäure (Phosphato-, Sulfato- und Thiosulfatogruppen), ebenso Dialkylaminogruppen mit Alkylgruppen von jeweils 1 bis 4 C-Atomen, wie Dimethylamino und Diethylamino. Bevorzugt sind Y und $Y^1$ β-Chlorethyl, β-Sulfatoethyl oder Vinyl.

Die Gruppen "Sulfo", "Carboxy", "Thiosulfato", "Phosphato" und "Sulfato" schließen sowohl deren Säureform als auch deren Salzform ein. Demgemäß bedeuten Sulfogruppen Gruppen entsprechend der allgemeinen Formel $-SO_3M$ , Carboxygruppen Gruppen entsprechend der allgemeinen Formel -COOM, Thiosulfatogruppen Gruppen entsprechend der allgemeinen Formel $-S-SO_3M$ , Phosphatogruppen Gruppen entsprechend der allgemeinen Formel $-OPO_3M_2$ und Sulfatogruppen Gruppen entsprechend der allgemeinen Formel $-OSO_3M$ , in welchen M die obengenannte Bedeutung besitzt.

Reste der allgemeinen Formel (2a) und (2b) sind beispielsweise 2-Sulfo-4-(β-sulfatoethylsulfonyl)-phenyl, 2'-Sulfo-5-(β-sulfatoethylsulfonyl)-phenyl, 2-Sulfo-4-vinylsulfonyl-phenyl, 6-Sulfo-8-(β-sulfatoethylsulfonyl)-naphth-2-yl, 8-Sulfo-6-(β-sulfatoethylsulfonyl)-naphth-2-yl und 1-Sulfo-6-(β-sulfatoethylsulfonyl)-naphth-2-yl.

Reste der allgemeinen Formeln (3a) und (3b) sind beispielsweise β-(β'-Sulfatoethylsulfonyl)-ethylamino, β-(β'-Chlorethylsulfonyl)-ethylamino, γ-(β'-Sulfatoethylsulfonyl)-propylamino, γ-(β'-Chlorethylsulfonyl)-propylamino, β-(Vinylsulfonyl)-ethylamino, γ-(Vinylsulfonyl)-propylamino, N-Phenyl-N-[β-(β'-sulfatoethylsulfonyl)-ethyl]-amino, N-Phenyl-N-[β-(β'-chlorethylsulfonyl)-ethyl]-amino, N-Phenyl-N-[γ-(β'-chlorpropylsulfonyl)-propyl]-amino, N-Phenyl-N-[γ-(β'-sulfatopropylsulfonyl)-propyl]-amino, N-(4'-Sulfo-phenyl)-N-[β-(β'-sulfatoethylsulfonyl)-ethyl]-amino, N-(4'-Sulfo-phenyl)-N-[β-(β'-chlorethylsulfonyl)-ethyl]-amino, N-(4'-Sulfo-phenyl)-N-[γ-(β'-chlorethylsulfonyl)-propyl]-amino, N-(4'-Sulfo-phenyl)-N-[γ-(β'-sulfatoethylsulfonyl)-propyl]-amino. Bis-N,N-[β-(β'-chlorethylsulfonyl)-ethyl]-amino, Bis-N,N-[γ-(β'-chlorethylsulfonyl)-propyl]-amino,Bis-(β-vinylsulfonyl-ethyl)-amino, N-(3'-Sulfo-phenyl)-N-[β-(β'-sulfatoethylsulfonyl)-ethyl]-amino, N-(3'-Sulfo-phenyl)-N-[β-(β'-chlorethylsulfonyl)-ethyl]-amino, N-(3'-Sulfo-phenyl)-N-[γ-(β'-chlorethylsulfonyl)-propyl]-amino und N-(3'-Sulfo-phenyl)-N-[γ-(β'-sulfatoethylsulfonyl)-propyl]-amino.

Reste der allgemeinen Formel (3c) und (3d) sind beispielsweise 2-(β-Sulfatoethylsulfonyl)-phenyl, 3-(β-Sulfatoethylsulfonyl)-phenyl, 4-(β-Sulfatoethylsulfonyl)-phenyl, 2-Carboxy-5-(β-sulfatoethylsulfonyl)-phenyl, 2-Chlor-3-(sulfatoethylsulfonyl)-phenyl, 2-Chlor-4-(β-sulfatoethylsulfonyl)-phenyl, 2-Brom-4-(β-sulfatoethylsulfonyl)-phenyl, 4-Methoxy-3-(β-sulfatoethylsulfonyl)-phenyl, 4-Chlor-3-(β-sulfatoethylsulfonyl)-phenyl, 2-Ethoxy-4- oder -5-(β-sulfatoethylsulfonyl)-phenyl, 2-Methyl-4-(β-sulfatoethylsulfonyl)-phenyl, 2-Methoxy-5- oder -4-(β-sulfatoethylsulfonyl)-phenyl, 2,4-Diethoxy-5-(β-sulfatoethylsulfonyl)-phenyl, 2,4-Dimethoxy-5-(β-sulfatoethylsulfonyl)-phenyl, 2,5-Dimethoxy-4-(β-sulfatoethylsulfonyl)-phenyl, 2-Methoxy-5-methyl-4-(β-sulfatoethylsulfonyl)-phenyl, 2- oder 3- oder 4-(β-Thiosulfatoethylsulfonyl)-phenyl, 2-Methoxy-5-(β-thiosulfatoethylsulfonyl)-phenyl, 2-Sulfo-4-(β-phosphatoethylsulfonyl)-phenyl, 2-Sulfo-4-vinylsulfonyl-phenyl, 2-Hydroxy-4- oder -5-(β-sulfatoethylsulfonyl)-phenyl, 2-Chlor-4- oder -5-(β-chlorethylsulfonyl)-phenyl, 2-Hydroxy-5-(β-sulfatoethylsulfonyl)-phenyl, 2-Hydroxy-3-sulfo-5-(β-sulfatoethylsulfonyl)-phenyl, 3- oder 4-(β-Acetoxyethylsulfonyl)-phenyl, 6-Carboxy-1-sulfo-naphth-2-yl, 5-(β-Sulfatoethylsulfonyl)-naphth-2-yl, 6- oder 7- oder 8-(β-Sulfatoethylsulfonyl)-naphth-2-yl, 6-(β-Sulfatoethylsulfonyl)-1-sulfo-naphth-2-yl, 5-(β-Sulfatoethylsulfonyl)-1-sulfo-naphth-2-yl und 8-(β-Sulfatoethylsulfonyl)-6-sulfo-naphth-2-yl.

Die vorliegende Erfindung betrifft weiterhin ein Verfahren zur Herstellung der erfindungsgemäßen Disazoverbindungen der allgemeinen Formel (1), das dadurch gekennzeichnet ist, daß man eine Diazoniumverbindung eines Amins der allgemeinen Formel $D-NH_2$ mit D der obengenannten Bedeutung oder die Diazoniumverbindungen zweier Amine der allgemeinen Formel $D-NH_2$ in der zweifach äquivalenten Menge mit einer Verbindung der Formel (4)

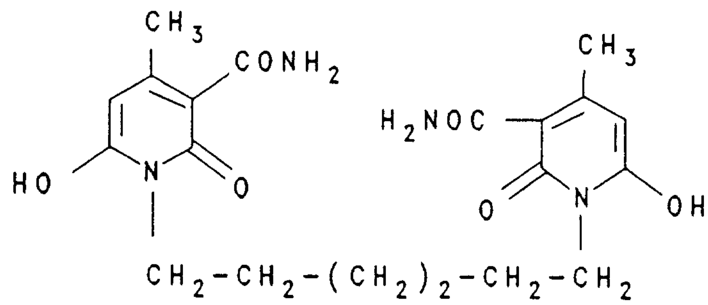

(4)

bei einer Temperatur zwischen 0 und 50 °C, bevorzugt zwischen 15 und 20 °C, und bei einem pH-Wert zwischen 3 und 7, bevorzugt zwischen 4 und 6,5, umsetzt.

Die Kupplungsreaktion erfolgt bevorzugt im wäßrigen Medium; sie kann auch in wäßrig-organischem Medium durchgeführt werden, wobei der organische Lösemittelanteil Aceton, Dimethylsulfoxid, Dimethylformamid oder N-Methyl-pyrrolidin sein kann.

Die Herstellung der Diazoniumverbindungen des Amins D-$NH_2$ erfolgt in üblicher Weise der Diazotierung von aromatischen Aminoverbindungen, so beispielsweise in wäßrigem Medium bei einem pH-Wert von kleiner als 2 mittels salpetriger Säure (Natriumnitrit) bei einer Temperatur zwischen -5 °C und + 15 °C.

Die zur Synthese der erfindungsgemäßen Disazoverbindungen der allgemeinen Formel (1) einsetzbaren Ausgangsverbindungen sind, außer der Ausgangsverbindung der Formel (4), zahlreich aus veröffentlichten Druckschriften, faserreaktive Farbstoffe betreffend, bekannt oder können analog den darin gemachten Angaben synthetisiert werden.

Die Ausgangsverbindung der Formel (4) ist neu. Die vorliegende Erfindung betrifft somit auch diese neue Verbindung sowie Verfahren zu deren Herstellung und deren Verwendung zur Synthese von faserreaktiven Azofarbstoffen, insbesondere von Azofarbstoffen der allgemeinen Formel (1). Die Verbindung der allgemeinen Formel (4) läßt sich erfindungsgemäß herstellen, indem man N,N'-Di-(cyanoacetyl)-1,6-diaminohexan (bekannt aus der europäischen Patentanmeldungs-Veröffentlichung Nr. 0 226 541) mit Acetessigsäureethylester umsetzt und in der erhaltenen Verbindung anschließend die beiden Cyanogruppen zu den Carbamoylgruppen hydrolysiert. Die erfindungsgemäße Umsetzung erfolgt in der Regel in wäßrigem Medium in Gegenwart eines aliphatischen Amins als Hilfsbase, wie beispielsweise Methylamin, Ethylamin, Propylamin, Butylamin, Ethylendiamin oder Hexylendiamin, insbesondere Hexylendiamin, unter Rückflußtemperatur. Die Hydrolyse erfolgt im allgemeinen mittels einer anorganischen starken Säure, wie beispielsweise Salzsäure oder Schwefelsäure, insbesondere Schwefelsäure, bei einer Temperatur zwischen 60 und 120 °C, bevorzugt zwischen 80 und 100 °C.

Die Abscheidung und Isolierung der erfindungsgemäß hergestellten Verbindungen der allgemeinen Formel (1) aus den Syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei dieser Reaktionslösung eine Puffersubstanz zugefügt werden kann.

Die erfindungsgemäßen Verbindungen der allgemeinen Formel (1) haben faserreaktive Eigenschaften und besitzen sehr gute Farbstoffeigenschaften. Sie können deshalb zum Färben (einschließlich Bedrucken) von hydroxygruppenhaltigen und/oder carbonamidgruppenhaltigen Materialien verwendet werden. Auch können die bei der Synthese der erfindungsgemäßen Verbindungen anfallenden Lösungen, gegebenenfalls nach Zusatz einer Puffersubstanz, gegebenenfalls auch nach Konzentrierung, direkt als Flüssigpräparation der färberischen Verwendung zugeführt werden.

Gegenstand der vorliegenden Erfindung ist deshalb auch die Verwendung der Verbindungen (1) zum Färben von hydroxy- und carbonamidgruppenhaltigen Materialien bzw. Verfahren zum Färben solcher Materialien, bei welchem man die Verbindungen (1) auf das Material aufbringt oder in das Material einbringt und sie auf oder in dem Material mittels Wärme oder mit Hilfe eines alkalisch wirkenden Mittels oder mittels beider Maßnahmen fixiert. Eingeschlossen sind hierbei die Massefärbung, beispielsweise Folien aus Polyamid, und die Druckfärbung. Bevorzugt kommen die Materialien in Form von Fasermaterialien zur Anwendung, insbesondere in Form von Textilfasern, wie in Form von Geweben und Garnen, beispielsweise in Form von Strängen und Wickelkörpern.

Hydroxygruppenhaltige Materialien sind natürliche oder synthetische hydroxygruppenhaltige Materialien, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute

und Ramiefasern; regenerierte Cellulosefasern sind beispielsweise Zellwolle und viskose Kunstseide.

Carbonamidgruppenhaltige Materialien sind beispielsweise synthetische und natürliche Polyamide und Polyurethane, insbesondere in Form der Fasern, beispielsweise Wolle und andere Tierhaare, Seide, Leder, Polyamid-6,6, Polyamid-6, Polyamid-11 und Polyamid-4.

Die Verbindungen (1) lassen sich auf den genannten Substraten, insbesondere auf den genannten Fasermaterialien, nach den für wasserlösliche Farbstoffe, insbesondere für faserreaktive Farbstoffe, bekannten Anwendungstechniken applizieren und fixieren. So erhält man mit ihnen auf Cellulosefasern nach dem Ausziehverfahren aus langer Flotte unter Verwendung von verschiedensten säurebindenden Mitteln und gegebenenfalls neutralen Salzen, wie Natriumchlorid oder Natriumsulfat, Färbungen mit sehr guten Farbausbeuten sowie ausgezeichnetem Farbaufbau bei hohen Fixiergraden. Man färbt bei Temperaturen zwischen 40 und 105 °C, gegebenenfalls bei Temperaturen bis 120 °C unter Druck, und gegebenenfalls in Gegenwart von üblichen Färbereihilfsmitteln im wäßrigen Bad. Man kann dabei so vorgehen, daß man das Material in das warme Bad einbringt und es allmählich auf die gewünschte Färbetemperatur erwärmt und den Färbeprozeß bei dieser Temperatur zu Ende führt. Die das Ausziehen der Verbindungen (1) beschleunigenden Neutralsalze können dem Bad gewünschtenfalls auch erst nach Erreichen der eigentlichen Färbetemperatur zugesetzt werden.

Nach dem Klotzverfahren werden auf Cellulosefasern ebenfalls ausgezeichnete Farbausbeuten mit hohen Fixiergraden und ein sehr guter Farbaufbau erhalten, wobei durch Verweilen bei Raumtemperatur oder erhöhter Temperatur, beispielsweise bis zu etwa 60 °C, durch Dämpfen oder mit Trockenhitze in üblicher Weise fixiert werden kann.

Ebenfalls erhält man nach den üblichen Druckverfahren für Cellulosefasern, die entweder einphasig durchgeführt werden können, beispielsweise durch Bedrucken mit einer Natriumcarbonat oder ein anderes säurebindendes Mittel und die Verbindung (1) enthaltenden Druckpaste und durch anschließendes Dämpfen bei 100 bis 103 °C, oder die zweiphasig, beispielsweise durch Bedrucken mit neutraler oder schwach saurer, das Farbmittel enthaltenden Druckpaste und anschließendes Fixieren entweder durch Hindurchführen der bedruckten Ware durch ein heißes elektrolythaltiges Bad oder durch Überklotzen mit einer alkalischen elektrolythaltigen Klotzflotte mit anschließendem Verweilen dieses behandelten Materials oder anschließendem Dämpfen oder anschließender Behandlung mit Trockenhitze, durchgeführt werden können, farbstarke Drucke mit gutem Stand der Konturen. Der Ausfall der Drucke ist von wechselnden Fixierbedingungen nur wenig abhängig. Sowohl in der Färberei als auch in der Druckerei sind die mit den Verbindungen (1) erhaltenen Fixiergrade sehr hoch.

Bei der Fixierung mittels Trockenhitze nach den üblichen Thermofixierverfahren verwendet man Heißluft von 120 bis 200 °C. Neben dem üblichen Wasserdampf von 101 bis 103 °C kann auch überhitzter Dampf und Druckdampf von Temperaturen bis 160 °C eingesetzt werden.

Die säurebindenden und die Fixierung der Verbindungen (1) auf den Cellulosefasern bewirkenden Mittel sind beispielsweise wasserlösliche basische Salze der Alkalimetalle und der Erdalkalimetalle von anorganischen oder organischen Säuren, ebenso Verbindungen, die in der Hitze Alkali freisetzen. Insbesondere sind die Alkalimetallhydroxide und Alkalimetallsalze von schwachen bis mittelstarken anorganischen oder organischen Säuren zu nennen, wobei von den Alkaliverbindungen vorzugsweise die Natrium- und Kaliumverbindungen gemeint sind. Solche säurebindenden Mittel sind beispielsweise Natriumhydroxid, Kaliumhydroxid, Natriumcarbonat, Natriumhydrogencarbonat, Kaliumcarbonat, Natriumformiat, Natriumdihydrogenphosphat, Dinatriumhydrogenphosphat, Natriumtrichloracetat, Wasserglas oder Trinatriumphosphat.

Durch die Behandlung der Verbindungen (1) mit den säurebindenden Mitteln, gegebenenfalls unter Wärmeeinwirkung, werden diese chemische an die Cellulosefaser gebunden; insbesondere die Cellulosefärbungen zeigen nach der üblichen Nachbehandlung durch Spülen zur Entfernung von nicht fixierten Anteilen der Verbindungen (1) ausgezeichnete Naßechtheiten, zumal sich solche nicht fixierten Anteile leicht wegen ihrer guten Kaltwasserlöslichkeit auswaschen lassen.

Die Färbungen auf Polyurethan- und Polyamidfasern werden üblicherweise aus saurem Milieu ausgeführt. So kann man beispielsweise dem Färbebad Essigsäure und/oder Ammoniumsulfat und/oder Essigsäure und Ammoniumacetat oder Natriumacetat zufügen, um den gewünschten pH-Wert zu erhalten. Zwecks Erreichung einer brauchbaren Egalität der Färbung empfiehlt sich ein Zusatz an üblichen Egalisierungshilfsmitteln, wie beispielsweise auf Basis eines Umsetzungsproduktes von Cyanurchlorid mit der dreifachen molaren Menge einer Aminobenzolsulfonsäure und/oder einer Aminonaphthalinsulfonsäure oder auf Basis eines Umsetzungsproduktes von beispielsweise Stearylamin mit Ethylenoxid. In der Regel wird das zu färbende Material bei einer Temperatur von etwa 40 °C in das Bad eingebracht, dort einige Zeit darin bewegt, das Färbebad dann auf den gewünschten schwach sauren, vorzugsweise schwach essigsauren, pH-Wert nachgestellt und die eigentliche Färbung bei einer Temperatur zwischen 60 und 98 °C durchgeführt. Die Färbungen können aber auch bei Siedetemperaturen oder bei Temperaturen bis zu

120 ° C (unter Druck) ausgeführt werden.

Die mit den Verbindungen (1) hergestellten Färbungen und Drucke zeichnen sich durch klare Nuancen aus. Insbesondere die Färbungen und Drucke auf Cellulosematerial besitzen, wie bereits erwähnt, darüber hinaus eine hohe Farbstärke, eine gute Lichtechtheit und gute Naßechtheiten, wie Wasch-, Walk-, Wasser-, Überfärbe- und Schweißechtheiten, des weiteren eine gute Plissierechtheit, Bügelechtheit und Reibechtheit.

Besonders hervorzuheben sind die mit den erfindungsgemäßen Farbstoffen auf Cellulosefasermaterialien erzielbaren hohen Fixierausbeuten, die bei der Anwendung nach Druckverfahren und Klotzfärbeverfahren über 90% betragen können. Ein weiterer Vorteil der Verbindungen (1) besteht in der leichten Auswaschbarkeit der beim Druck- oder Färbevorgang nicht fixierten Anteile, wodurch der Waschvorgang der bedruckten oder gefärbten Cellulosefasermaterialien mit geringen Waschflottenmengen und gegebenenfalls einer energiesparenden Temperaturführung während des Waschvorganges bewerkstelligt werden kann.

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säure angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Farbstoffe angegebenen Absorptionsmaxima ($\lambda_{max}$) im sichtbaren Bereich wurden anhand ihrer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

Beispiel A

Eine Mischung aus 25 Teilen N,N'-Di-cyanoacetyl-1,6-diamino-hexan, 28,4 Teilen Acetessigsäureethylester und 29 Teilen Hexylendiamin in 100 Teilen Wasser wird vier Stunden unter Rückfluß erhitzt. Das Reaktionsgemisch wird danach abgekühlt und in eine Mischung aus 100 Teilen konzentrierter wäßriger Salzsäure und 100 Teilen Eis eingerührt. Man erhält einen weißen Niederschlag, der abgesaugt, mit Wasser gewaschen, von Wasser gut abgesaugt und anschließend in 60 Teile konzentrierte wäßrige Schwefelsäure eingetragen wird. Die Mischung erhitzt man während drei Stunden bei 90 ° C und rührt sie sodann in ein Gemisch aus 60 Teilen Eis und 6 Teilen konzentrierter Schwefelsäure ein, wobei man durch Zugabe von weiterem Eis die Temperatur unter 30 ° C hält. Man rührt noch etwa 30 Minuten nach und saugt das ausgefallene Produkt ab, wäscht es mit Wasser und trocknet es.

Die so erhaltene Verbindung der Formel (4) besitzt einen Schmelzpunkt von 183 bis 185 ° C (unter Zersetzung).

Sie besitzt folgende Banden im IR-Spektrum (in $cm^{-1}$):

3515 (NH), 3353 (NH/OH), 2966 (CH), 2938 (CH), 1637 (CO), 1567 (CO).

Beispiel 1

Eine Suspension von 18,4 Teilen Cyanurchlorid in 100 Teilen Eis und 200 Teilen Wasser versetzt man unter gutem Rühren mit einer Lösung mit einem pH-Wert von 4,5 von 28,1 Teilen 4-(β-Sulfatoethylsulfonyl)-anilin in 200 Teilen Wasser. Man rührt das Reaktionsgemisch noch 30 Minuten bei einem pH-Wert von 4,0 bis 4,5 und einer Temperatur von 5 bis 10 ° C weiter, erwärmt den Ansatz sodann innerhalb von 30 Minuten auf 20 bis 25 ° C, rührt noch etwa eine Stunde nach, versetzt die erhaltene Suspension mit einer Lösung mit einem pH-Wert von 6 von 18,8 Teilen 1,3-Diaminobenzol-4-sulfonsäure in 200 Teilen Wasser und rührt das Reaktionsgemisch etwa zwei Stunden bei einem pH-Wert von 6 und bei einer Temperatur von 20 bis 25 ° C nach. Der Ansatz wird sodann auf 0 bis 3 ° C abgekühlt, mit konzentrierter wäßriger Salzsäure auf einen pH-Wert von 1,2 gestellt und die erhaltene Aminoverbindung durch Einrühren von 20 Teilen einer wäßrigen 5N-Natriumnitritlösung in üblicher Weise diazotiert. Nach Entfernen überschüssiger salpetriger Säure gibt man 20 Teile der Verbindung der Formel (4) hinzu und führt die Kupplungsreaktion bei 15 bis 20 ° C und bei einem pH-Wert von 5,5 bis 6 durch.

Man isoliert die erhaltene erfindungsgemäße Pyridon-Disazoverbindung, die, in Form der freien Säure geschrieben, die Formel

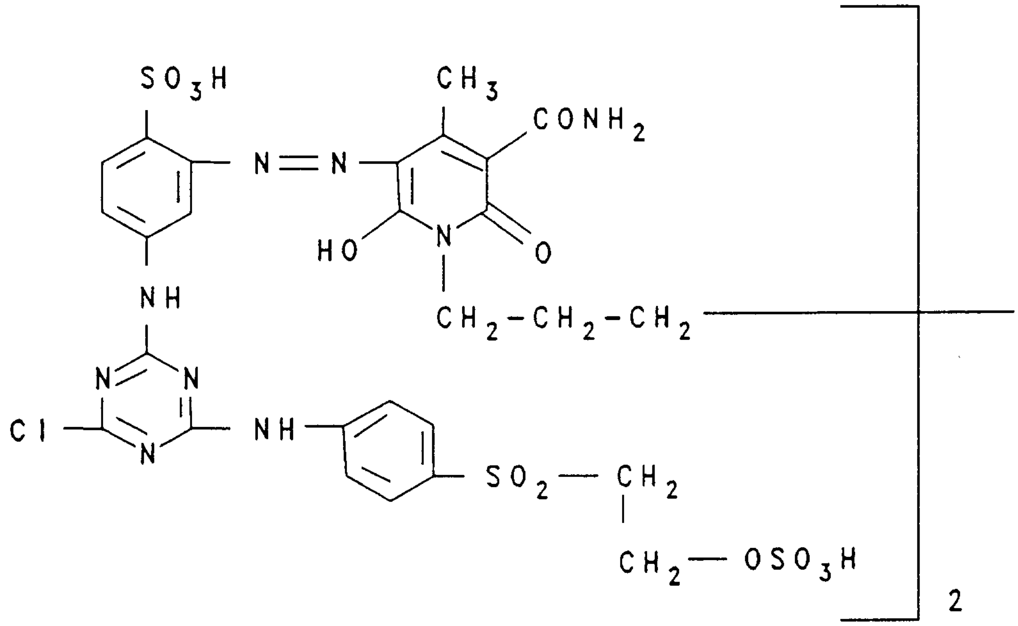

($\lambda_{max}$ = 422 nm)

besitzt, in üblicher Weise durch Eindampfen der Syntheselösung als elektrolytsalzhaltiges (natriumchlorid- und natriumsulfathaltiges) Alkalimetallsalz (Natriumsalz), das sehr gute faserreaktive Farbstoffeigenschaften zeigt und nach den in der Technik üblichen Färbe- und Druckverfahren auf den in der Beschreibung genannten Materialien, wie insbesondere Cellulosefasermaterialien, wie beispielsweise Baumwolle, farbstarke grünstichig gelbe Färbungen und Drucke mit guten Echtheitseigenschaften, von denen insbesondere die gute Lichtechtheit hervorgehoben werden kann, liefert.

Beispiel 2

Man rührt eine Lösung mit einem pH-Wert von 6 von 26,8 Teilen 1,3-Diaminobenzol-4,6-disulfonsäure in 200 Teilen Wasser in eine Suspension von 18,4 Teilen Cyanurchlorid in 100 Teilen Eis und 200 Teilen Wasser ein, rührt das Reaktionsgemisch noch eine Stunde bei einem pH-Wert von 2,0 und einer Temperatur von 5 bis 10 °C und rührt danach den Reaktionsansatz in einer Lösung mit einem pH-Wert von 6 von 28,1 Teilen 4-(β-Sulfatoethylsulfonyl)-anilin in 200 Teilen Wasser ein. Man stellt den pH-Wert des Ansatzes auf 4,0 bis 4,5, erwärmt ihn auf 40 bis 45 °C und führt die Umsetzung unter Rühren noch etwa 90 Minuten bei diesem pH- und Temperaturbereich weiter. Anschließend wird der Ansatz auf 0 bis 3 °C abgekühlt und mit konzentrierter wäßriger Salzsäure auf einen pH-Wert von 1,2 eingestellt. Die anschließende Diazotierung der erhaltenen Aminoverbindung und deren Kupplung mit der Verbindung (4) erfolgt in der im Beispiel 1 angegebenen Verfahrensweise.

Die erfindungsgemäße Disazoverbindung wird in üblicher Weise als Alkalimetallsalz (Natriumsalz) isoliert. Sie besitzt, in Form der freien Säure geschrieben, die Formel

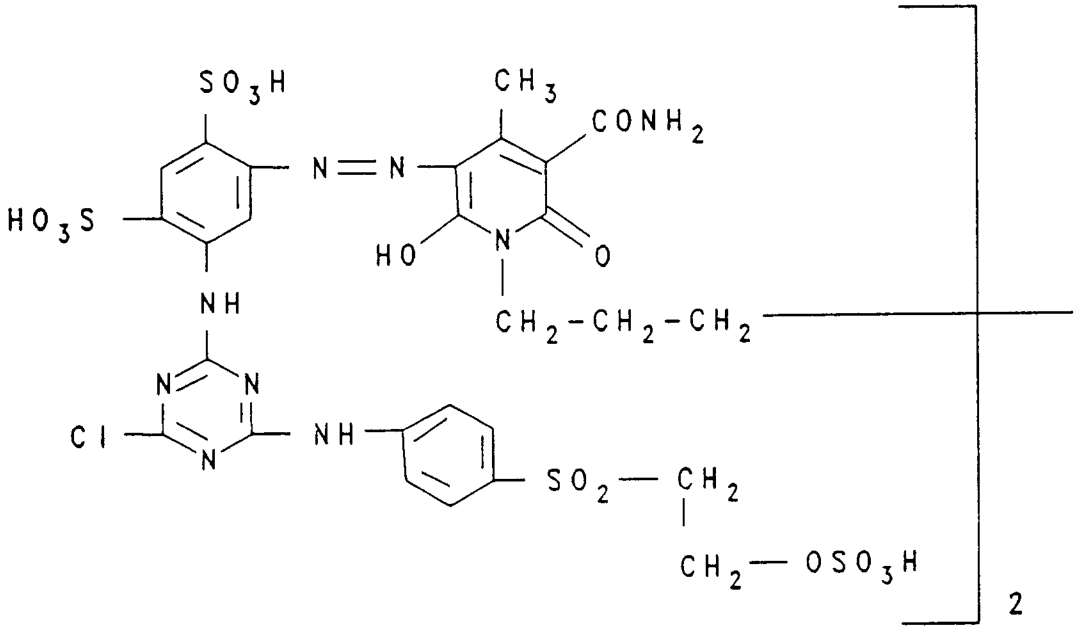

($\lambda_{max}$ = 421 nm)
besitzt sehr gute faserreaktive Farbstoffeigenschaften und liefert nach den in der Technik für faserreaktive Farbstoffe üblichen Anwendungsbedingungen auf den in der Beschreibung genannten Materialien, wie insbesondere Cellulosefasermaterialien, wie beispielsweise Baumwolle, farbstarke grünstichig gelbe Färbungen und Drucke mit guten Echtheitseigenschaften, von denen insbesondere die gute Lichtechtheit hervorgehoben werden kann.

Beispiel 3

Man rührt bei etwa 0 bis 3°C 4,3 Teile Cyanamid in eine Suspension aus 18,4 Teilen Cyanurchlorid in 200 Teilen Wasser und 200 Teilen Eis ein, stellt sodann mittels konzentrierter wäßriger Natronlauge einen pH-Wert von 9 ein und führt die Umsetzung während zwei Stunden bei 0 bis 3°C und einem pH-Wert von 9 durch. Anschließend stellt man mit verdünnter wäßriger Salzsäure einen pH-Wert von 5 ein und gibt 29,5 Teile 4-($\beta$-Sulfatoethylsulfonyl)-anilin hinzu, rührt noch etwa vier Stunden bei einer Temperatur von 5 bis 10°C weiter, wobei man den pH-Wert bis auf 1,5 bis 1 abfallen läßt, stellt sodann den Ansatz mit Natriumcarbonat auf einen pH-Wert von 5,5 und gibt danach die erhaltene Lösung zu einer Lösung aus 18,8 Teilen 1,3-Diaminobenzol-4-sulfonsäure in 600 Teilen Wasser. Der pH-Wert des Ansatzes wird auf 3,5 gestellt und der Ansatz unter Einhaltung des pH-Wertes während zwei Stunden bei 80°C erhitzt. Das Reaktionsgemisch wird anschließend auf 0 bis 3°C abgekühlt, konzentrierte wäßrige Salzsäure bis zu einem pH-Wert von 1,2 hinzugegeben und die in Lösung befindliche, erhaltene Diaminoverbindung gemäß den Angaben des Beispieles 1 diazotiert und mit der Pyridonverbindung der Formel (4) gekuppelt.

Man isoliert die erfindungsgemäße Disazoverbindung aus der Syntheselösung in üblicher Weise als Alkalimetallsalz (Natriumsalz), beispielsweise durch Sprühtrocknung der Syntheselösung. Sie besitzt, in Form der freien Säure geschrieben, die Formel

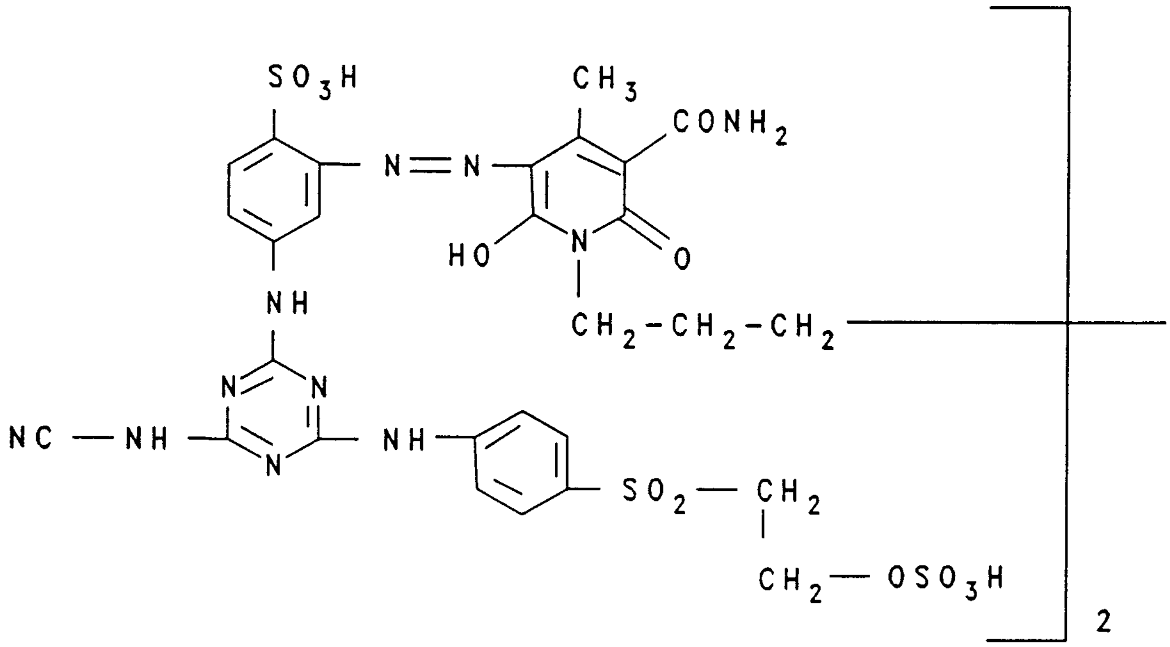

($\lambda_{max}$ = 422 nm)
und färbt als Verbindung mit guten faserreaktiven Farbstoffeigenschaften, beispielsweise Cellulosefasermaterialien, wie Baumwolle, nach den für faserreaktive Farbstoffe üblichen Applikations- und Fixierverfahren in farbstarken, grünstichig gelben Nuancen mit guten Echtheitseigenschaften, von denen insbesondere die gute Lichtechtheit hervorgehoben werden kann.

Beispiel 4

Man löst 14,2 Teile Dinatriumhydrogenphosphat in einer Lösung mit einem pH-Wert von 6,5 und einer Temperatur von 0 bis 3 °C von 17,3 Teilen Anilin-2-sulfonsäure in 600 Teilen Wasser und gibt sodann unter gutem Rührem innerhalb von etwa fünf Minuten langsam und stetig 18,4 Teile Cyanurfluorid hinzu. Der gebildete weiße Niederschlag wird anschließend abgesaugt und in 300 Teilen Wasser von 0 bis 3 °C suspendiert. Diese Suspension wird mit 11,3 Teilen 1,3-Diaminobenzol-4-sulfonsäure versetzt, und das Reaktionsgemisch wird etwa eine Stunde unter Einhaltung eines pH-Wertes von 5,5 gerührt; während der Umsetzung erwärmt man den Ansatz auf 20 bis 25 °C. Danach wird er wieder auf 0 bis 3 °C abgekühlt und mit konzentrierter wäßriger Salzsäure auf einen pH-Wert von 1,2 gestellt. Die Diazotierung der erhaltenen Diaminoverbindung und deren Kupplung mit der Pyridonverbindung der Formel (4) erfolgt in der im Beispiel 1 angegebenen Verfahrensweise.

Man isoliert die erfindungsgemäße Disazoverbindung aus der Syntheselösung, beispielsweise durch Sprühtrocknung. Sie besitzt, in Form der freien Säure geschrieben, die Formel

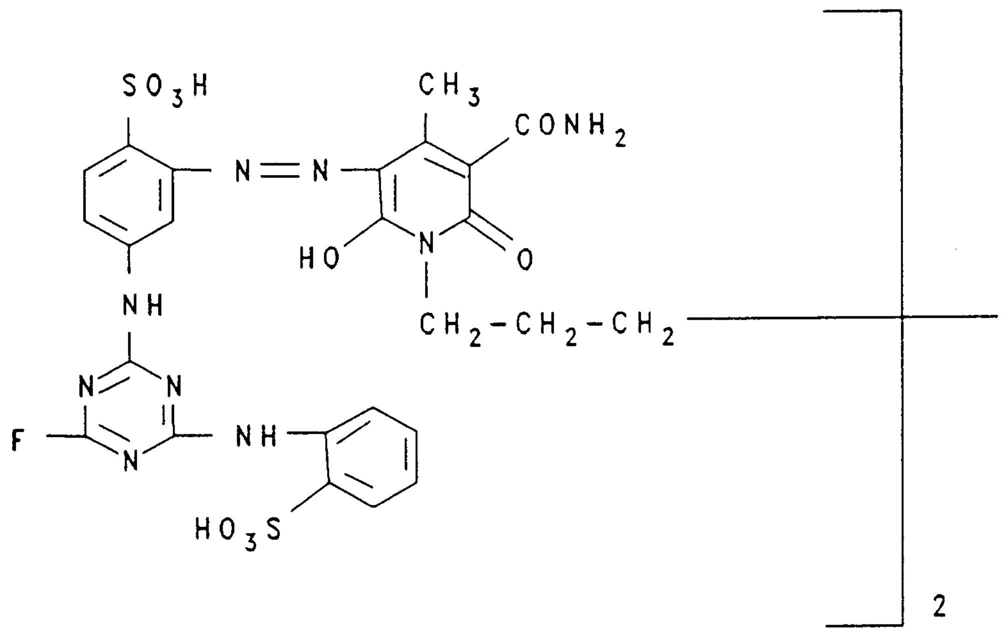

($\lambda_{max}$ = 422 nm)
zeigt sehr gute faserreaktive Farbstoffeigenschaften und färbt nach den in der Technik für faserreaktive Farbstoffe üblichen Färbe- und Druckverfahren die in der Beschreibung genannten Materialien, wie insbesondere Cellulosefasermaterialien, in farbstarken grünstichig gelben Tönen mit guten Echtheitseigenschaften, von denen insbesondere die gute Lichtechtheit hervorgehoben werden kann.

Beispiele 5 bis 77

In den nachfolgenden Tabellenbeispielen werden weitere erfindungsgemäße Pyridon-Disazoverbindungen entsprechend der allgemeinen Formel (A)

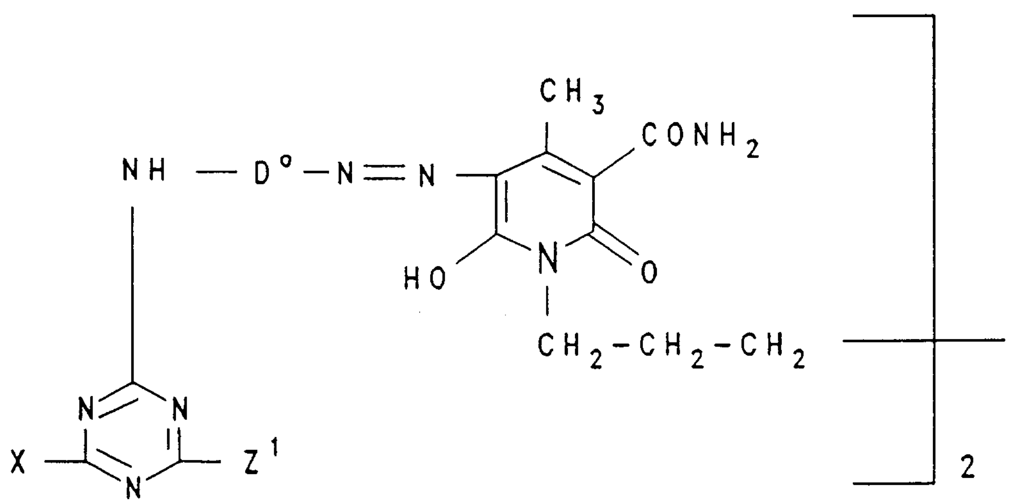

(A)

anhand ihrer Komponenten beschrieben. Sie lassen sich in erfindungsgemäßer Weise aus den aus der allgemeinen Formel (A) ersichtlichen Ausgangsverbindungen (der Diaminobenzolsulfonsäure entsprechend der allgemeinen Formel $H_2N\text{-}D^o\text{-}NH_2$ , Cyanurchlorid oder Cyanurfluorid oder dem Umsetzungsprodukt aus Cyanurchlorid und Cyanamid, einer Verbindung der allgemeinen Formel H-$Z^1$ sowie der Kupplungskomponente der Formel (4)) herstellen. Sie besitzen sehr gute faserreaktive Farbstoffeigenschaften und färben die in der Beschreibung genannten Materialien, wie insbesondere Cellulosefasermaterialien, in dem in dem jeweiligen Tabellenbeispiel (hier für Baumwolle) angegebenen Farbton in hoher Farbstärke und mit guten

Echtheiten.

| Bsp. | Rest -$D^o$- | Rest X | Rest $Z^1$ | Farbton |
|---|---|---|---|---|
| 5 | 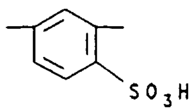 | Cyanoamino | 3-(ß-Sulfatoethyl-sulfonyl)-phenylamino | grünstichig gelb (420) |
| 6 | dito | Cyanoamino | 2-Methoxy-5-(ß-sulfatoethylsulfonyl)-phenylamino | grünstichig gelb (421) |
| 7 | dito | Cyanoamino | 4-(Vinylsulfonyl)-phenylamino | grünstichig gelb (422) |
| 8 | 4,6-Disulfo-1,3-phenylen | Cyanoamino | 3-(ß-Sulfatoethyl-sulfonyl)-phenylamino | grünstichig gelb |
| 9 | dito | Cyanoamino | 4-(ß-Sulfatoethyl-sulfonyl)-phenylamino | grünstichig gelb (423) |
| 10 | 2,5-Disulfo-1,4-phenylen | Cyanoamino | dito | rotstichig gelb (445) |
| 11 | 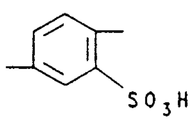 | Cyanoamino | dito | rotstichig gelb |
| 12 | dito | Cyanoamino | 3-(ß-Sulfatoethyl-sulfonyl)-phenylamino | rotstichig gelb |
| 13 | dito | Fluor | 2-Sulfo-phenylamino | rotstichig gelb (443) |
| 14 | dito | Fluor | 3-Sulfo-phenylamino | rotstichig gelb |
| 15 | dito | Fluor | 4-Sulfo-phenylamino | rotstichig gelb |

| Bsp. | Rest -D°- | Rest X | Rest $Z^1$ | Farbton |
|---|---|---|---|---|
| 16 | dito | Fluor | 4-(ß-Sulfatoethyl-sulfonyl)-phenylamino | rotstichig gelb (444) |
| 17 | dito | Fluor | 3-(ß-Sulfatoethyl-sulfonyl)-phenylamino | rotstichig gelb (442) |
| 18 | dito | Fluor | 2-Methoxy-5-(ß-sulfatoethylsulfonyl)-phenylamino | rotstichig gelb |
| 19 | dito | Fluor | 4-(Vinylsulfonyl)-phenylamino | rotstichig gelb |
| 20 | dito | Fluor | γ-(ß'-Sulfatoethyl-sulfonyl)-propylamino | rotstichig gelb |
| 21 | dito | Fluor | N-Phenyl-N-[ß-(ß'-sulfatoethylsulfonyl)-ethyl]-amino | rotstichig gelb (445) |
| 22 | dito | Fluor | N-Phenyl-N-[γ-(ß'-sulfatoethylsulfonyl)-propyl]-amino | rotstichig gelb (446) |
| 23 | dito | Fluor | γ-(ß'-Chlorethyl-sulfonyl)-propylamino | rotstichig gelb |
| 24 | dito | Fluor | γ-(Vinylsulfonyl)-propylamino | rotstichig gelb |
| 25 | dito | Fluor | Bis-N,N-(ß-vinyl-sulfonyl-ethyl)-amino | rotstichig gelb |
| 26 | dito | Fluor | Bis-N,N-[ß-(ß'-chlorethylsulfonyl)-ethyl]-amino | rotstichig gelb (445) |

| Bsp. | Rest -D°- | Rest X | Rest $Z^1$ | Farbton |
|---|---|---|---|---|
| 27 | dito | Fluor | Bis-N,N-[γ-(ß'-chlorethylsulfonyl)-propyl]-amino | rotstichig gelb |
| 28 | 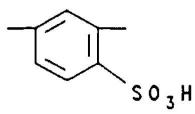 | Fluor | 3-Sulfo-phenylamino | grünstichig gelb (424) |
| 29 | dito | Fluor | 4-Sulfo-phenylamino | grünstichig gelb |
| 30 | dito | Fluor | 4-(ß-Sulfatoethyl-sulfonyl)-phenylamino | grünstichig gelb (422) |
| 31 | dito | Fluor | 4-(Vinylsulfonyl)-phenylamino | grünstichig gelb (422) |
| 32 | dito | Fluor | 3-(ß-Sulfatoethyl-sulfonyl)-phenylamino | grünstichig gelb |
| 33 | dito | Fluor | 2-Methoxy-5-(ß-sulfatoethylsulfonyl)-phenylamino | grünstichig gelb |
| 34 | dito | Fluor | γ-(ß'-Sulfatoethyl-sulfonyl)-propylamino | grünstichig gelb (424) |
| 35 | dito | Fluor | γ-(ß'-Chlorethyl-sulfonyl)-propylamino | grünstichig gelb |
| 36 | dito | Fluor | γ-(Vinylsulfonyl)-propylamino | grünstichig gelb |
| 37 | 2,5-Disulfo-1,4-phenylen | Fluor | 2-Sulfo-phenylamino | rotstichig gelb (443) |

| Bsp. | Rest -$D^o$- | Rest X | Rest $Z^1$ | Farbton |
|---|---|---|---|---|
| 38 | dito | Fluor | 3-Sulfo-phenylamino | rotstichig gelb (444) |
| 39 | dito | Fluor | 4-Sulfo-phenylamino | rotstichig gelb |
| 40 | dito | Fluor | 4-(ß-Sulfatoethyl-sulfonyl)-phenylamino | rotstichig gelb (445) |
| 41 | dito | Fluor | 3-(ß-Sulfatoethyl-sulfonyl)-phenylamino | rotstichig gelb |
| 42 | 4,6-Disulfo-1,3-phenylen | Fluor | 2-Sulfo-phenylamino | grünstichig gelb (423) |
| 43 | dito | Fluor | 3-Sulfo-phenylamino | grünstichig gelb |
| 44 | dito | Fluor | 4-Sulfo-phenylamino | grünstichig gelb |
| 45 | dito | Fluor | 4-(ß-Sulfatoethyl-sulfonyl)-phenylamino | grünstichig gelb (423) |
| 46 | dito | Fluor | 3-(ß-Sulfatoethyl-sulfonyl)-phenylamino | grünstichig gelb |
| 47 | dito | Fluor | 4-(Vinylsulfonyl)-phenylamino | grünstichig gelb |
| 48 | 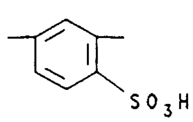 | Chlor | 2-Sulfo-phenylamino | grünstichig gelb (423) |
| 49 | dito | Chlor | 3-Sulfo-phenylamino | grünstichig gelb |

| Bsp. | Rest -D°- | Rest X | Rest $Z^1$ | Farbton |
|---|---|---|---|---|
| 50 | dito | Chlor | 4-Sulfo-phenylamino | grünstichig gelb |
| 51 | dito | Chlor | 3-(ß-Sulfatoethyl-sulfonyl)-phenylamino | grünstichig gelb (422) |
| 52 | dito | Chlor | 2-Methoxy-5-(ß-sulfatoethylsulfonyl)-phenylamino | grünstichig gelb |
| 53 | dito | Chlor | 4-(Vinylsulfonyl)-phenylamino | grünstichig gelb (422) |
| 54 | dito | Chlor | γ-(ß'-Chlorethyl-sulfonyl)-propyl-amino | grünstichig gelb (421) |
| 55 | dito | Chlor | γ-(Vinylsulfonyl)-propylamino | grünstichig gelb |
| 56 | dito | Chlor | N-Phenyl-N-[ß-(ß'-sulfatoethylsulfonyl)-ethyl]-amino | grünstichig gelb (423) |
| 57 | dito | Chlor | N-Phenyl-N-[γ-(ß'-sulfatoethylsulfonyl)-propyl]-amino | grünstichig gelb (422) |
| 58 | dito | Chlor | Bis-N,N-(ß-vinyl-sulfonylethyl)-amino | grünstichig gelb (422) |
| 59 | dito | Chlor | Bis-N,N-[ß-(ß'-chlor-ethylsulfonyl)-ethyl]-amino | grünstichig gelb (423) |

| Bsp. | Rest -D°- | Rest X | Rest $Z^1$ | Farbton |
|---|---|---|---|---|
| 60 | dito | Chlor | γ-(ß'-Sulfatoethyl-sulfonyl)-propylamino | grünstichig gelb |
| 61 | 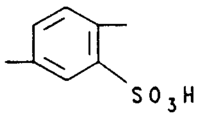 | Chlor | 2-Sulfo-phenylamino | rotstichig gelb (445) |
| 62 | dito | Chlor | 3-Sulfo-phenylamino | rotstichig gelb |
| 63 | dito | Chlor | 4-Sulfo-phenylamino | rotstichig gelb (444) |
| 64 | dito | Chlor | 4-(Vinylsulfonyl)-phenylamino | rotstichig gelb |
| 65 | dito | Chlor | 3-(ß-Sulfatoethyl-sulfonyl)-phenylamino | rotstichig gelb (443) |
| 66 | dito | Chlor | 2-Methoxy-5-(ß-sulfatoethylsulfonyl)-phenylamino | rotstichig gelb |
| 67 | dito | Chlor | γ-(ß'-Sulfatoethyl-sulfonyl)-propylamino | rotstichig gelb |
| 68 | 4,6-Disulfo-1,3-phenylen | Chlor | 2-Sulfo-phenylamino | grünstichig gelb |
| 69 | dito | Chlor | 3-Sulfo-phenylamino | grünstichig gelb |
| 70 | dito | Chlor | 4-Sulfo-phenylamino | grünstichig gelb (445) |

| Bsp. | Rest -D°- | Rest X | Rest $Z^1$ | Farbton |
|---|---|---|---|---|
| 71 | 4,6-Disulfo-1,3-phenylen | Chlor | 4-(Vinylsulfonyl)-phenylamino | grünstichig gelb (423) |
| 72 | dito | Chlor | 3-(ß-Sulfatoethyl-sulfonyl)-phenylamino | grünstichig gelb (422) |
| 73 | 2,5-Disulfo-1,4-phenylen | Chlor | 2-Sulfo-phenylamino | rotstichig gelb (444) |
| 74 | dito | Chlor | 3-Sulfo-phenylamino | rotstichig gelb |
| 75 | dito | Chlor | 4-Sulfo-phenylamino | rotstichig gelb |
| 76 | dito | Chlor | 4-(ß-Sulfatoethyl-sulfonyl)-phenylamino | rotstichig gelb (443) |
| 77 | dito | Chlor | 3-(ß-Sulfatoethyl-sulfonyl)-phenylamino | rotstichig gelb |

Beispiel 78

Zu einer Lösung von 0 bis 3°C aus 41,1 Teilen 1-Sulfo-6-(β-sulfatoethylsulfonyl)-2-amino-naphthalin und 20 Teilen einer wäßrigen 5N-Natriumnitritlösung in 300 Teilen Wasser läßt man unter kräftigem Rühren konzentrierte wäßrige Salzsäure zulaufen, bis ein pH-Wert von 1,2 eingestellt ist. Man rührt den Ansatz noch etwa zwei Stunden nach und entfernt überschüssige salpetrige Säure in üblicher Weise, beispielsweise mittels Amidosulfonsäure. Sodann gibt man 20 Teile der Verbindung der Formel (4) hinzu und führt die Kupplungsreaktion bei 15 bis 20°C und einem pH-Wert von 5,5 bis 6,0 durch.

Die erfindungsgemäße Pyridon-Disazoverbindung wird in üblicher Weise isoliert, beispielsweise durch Eindampfen der Syntheselösung. Sie besitzt, in Form der freien Säure geschrieben, die Formel

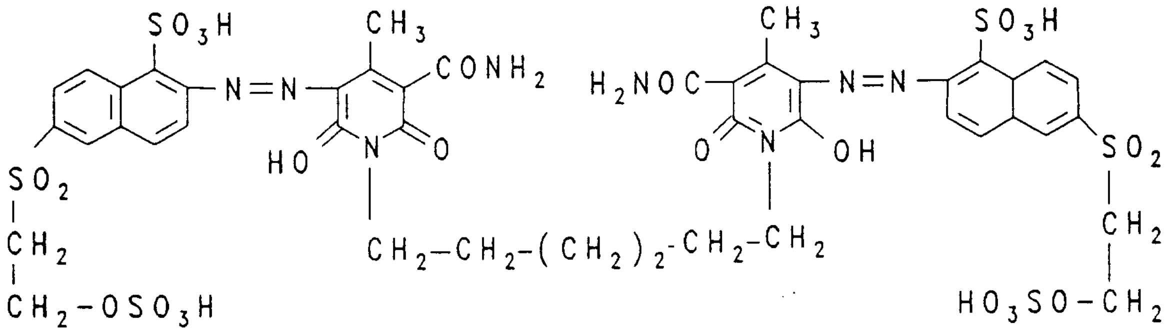

($\lambda_{max}$ = 430 nm)
und liefert nach den für faserreaktive Farbstoffe üblichen Färbe- und Druckverfahren auf den in der Beschreibung genannten Materialien, wie insbesondere Cellulosefasermaterialien, wie beispielsweise Baumwolle, farbstarke grünstichig gelbe Färbungen und Drucke mit guten Echtheitseigenschaften, von denen insbesondere die gute Lichtechtheit hervorgehoben werden kann.

Beispiele 79 bis 82

In den nachfolgenden Tabellenbeispielen werden weitere erfindungsgemäße Disazoverbindungen entsprechend der allgemeinen Formel (1) mit Hilfe des Restes D beschrieben. Sie lassen sich analog dem Beispiel 78 durch Diazotierung der entsprechenden, aus dem jeweiligen Tabellenbeispiel ersichtlichen aromatischen Aminoverbindung entsprechend der allgemeinen Formel D-$NH_2$ und nachfolgende Kupplung mit der Pyridonverbindung der Formel (4) herstellen. Sie besitzen sehr gute faserreaktive Farbstoffeigenschaften und färben die in der Beschreibung genannten Materialien, wie insbesondere Cellulosefasermaterialien, in farbstarken, echten Tönen in dem in dem jeweiligen Tabellenbeispiel angegebenen Farbton (hier auf Baumwolle).

| Bsp. | Rest D in Disazoverbindung der Formel (1) | Farbton |
|---|---|---|
| 79 | 8-Sulfo-6-(β-sulfatoethylsulfonyl)-naphth-2-yl | grünstichig gelb (428) |
| 80 | 6-Sulfo-8-(β-sulfatoethylsulfonyl)-naphth-2-yl | grünstichig gelb |
| 81 | 2-Sulfo-4-(β-sulfatoethylsulfonyl)-phenyl | grünstichig gelb |
| 82 | 2-Sulfo-5-(β-sulfatoethylsulfonyl)-phenyl | grünstichig gelb |

**Patentansprüche**

**1.** Pyridon-Disazoverbindung der allgemeinen Formel (1)

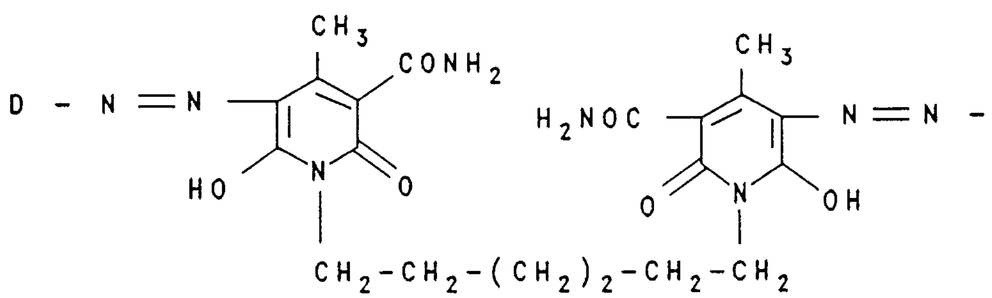

(1)

in welcher bedeuten:

D ist eine Gruppe der allgemeinen Formel (2a), (2b) oder (2c)

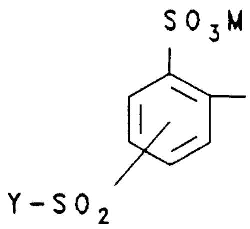 (2a)

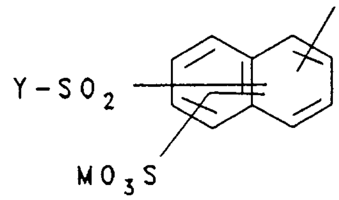 (2b)

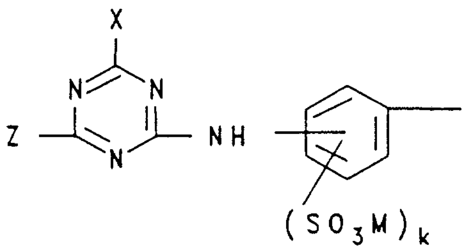 (2c)

in welchen

- Y Vinyl ist oder Ethyl ist, das in $\beta$-Stellung einen Substituenten enthält, der durch Alkali unter Bildung der Vinylgruppe eliminierbar ist,
- M Wasserstoff oder ein Alkalimetall ist,
- k die Zahl Null, 1 oder 2 ist (im Falle von k gleich Null steht diese Gruppe für ein Wasserstoffatom),
- X Chlor, Fluor, Hydroxy, Alkoxy von 1 bis 10 C-Atomen oder Cycloalkyloxy von 5 bis 8 C-Atomen ist oder Alkoxy von 3 bis 8 C-Atomen bedeutet, dessen Alkylrest durch eine oder zwei Heterogruppen unterbrochen ist, die aus den Gruppen -O- , -NH- , -CO-NH- und -NH-CO- ausgewählt sind, oder Benzyloxy, Sulfomethoxy, $\beta$-Sulfoethoxy, Amino, Alkylamino von 1 bis 10 C-Atomen oder Cycloalkylamino von 5 bis 8 C-Atomen ist oder Alkylamino von 3 bis 8 C-Atomen bedeutet, dessen Alkylrest durch eine oder zwei Heterogruppen unterbrochen ist, die aus den Gruppen -O- , -NH- , -CO-NH- und -NH-CO- ausgewählt sind, oder Benzylamino, Sulfomethylamino, $\beta$-Sulfoethylamino oder Cyanoamino oder Monosulfo-phenylamino, Disulfo-phenylamino, Monosulfo-naphth-2-ylamino, Disulfo-naphth-2-ylamino oder Trisulfo-naphth-2-ylamino ist und
- Z Chlor, Fluor oder eine Gruppe der allgemeinen Formel (3a), (3b), (3c) oder (3d)

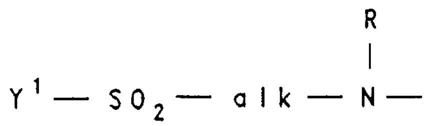

(3a)

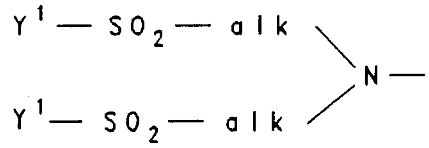

(3b)

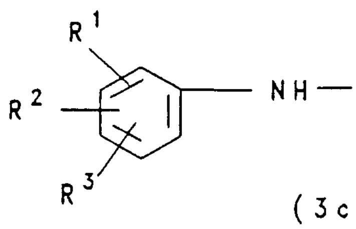

(3c)

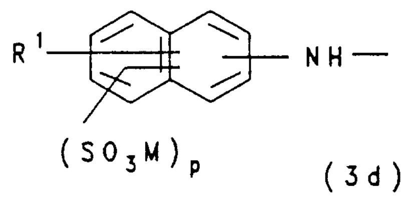

(3d)

ist, in welchen

- R Wasserstoff, Phenyl oder durch Sulfo, Ethyl, Ethoxy, Methyl oder Methoxy substituiertes Phenyl ist,
- alk Alkylen von 1 bis 8 C-Atomen bedeutet oder Alkylen von 3 bis 8 C-Atomen ist, das durch eine oder zwei Heterogruppen unterbrochen ist, die aus den Gruppen -O- , -NH- , -CO-NH- und -NH-CO- ausgewählt sind,
- $Y^1$ Vinyl ist oder Ethyl bedeutet, das in $\beta$-Stellung durch einen Substituenten substituiert ist, der durch Alkali unter Bildung der Vinylgruppe eliminierbar ist,
- $R^1$ Wasserstoff, Carboxy, Sulfo oder eine Gruppe der allgemeinen Formel $-SO_2-Y^1$ mit $Y^1$ der obengenannten Bedeutung ist,
- $R^2$ Wasserstoff, Alkyl von 1 bis 4 C-Atomen, Alkoxy von 1 bis 4 C-Atomen, Chlor, Brom, Carboxy, Sulfo oder Nitro ist,
- $R^3$ Wasserstoff, Alkyl von 1 bis 4 C-Atomen, Alkoxy von 1 bis 4 C-Atomen, Chlor, Brom, Carboxy oder Sulfo ist,
- M eine der obengenannten Bedeutungen besitzt und
- p für die Zahl 1 oder 2 steht.

**2.** Verbindung nach Anspruch 1, dadurch gekennzeichnet, daß X Chlor oder Fluor ist.

**3.** Verbindung nach Anspruch 1, dadurch gekennzeichnet, daß X Cyanoamino ist.

**4.** Verbindung nach mindestens einem der Ansprüche 1 bis 3, dadurch gekennzeichnet, daß Z eine Gruppe der allgemeinen Formel (3a) ist.

**5.** Verbindung nach mindestens einem der Ansprüche 1 bis 3, dadurch gekennzeichnet, daß Z eine Gruppe der allgemeinen Formel (3b) ist.

**6.** Verbindung nach mindestens einem der Ansprüche 1 bis 3, dadurch gekennzeichnet, daß Z eine Gruppe der allgemeinen Formel (3c) ist.

**7.** Verbindung nach Anspruch 1 der Formel

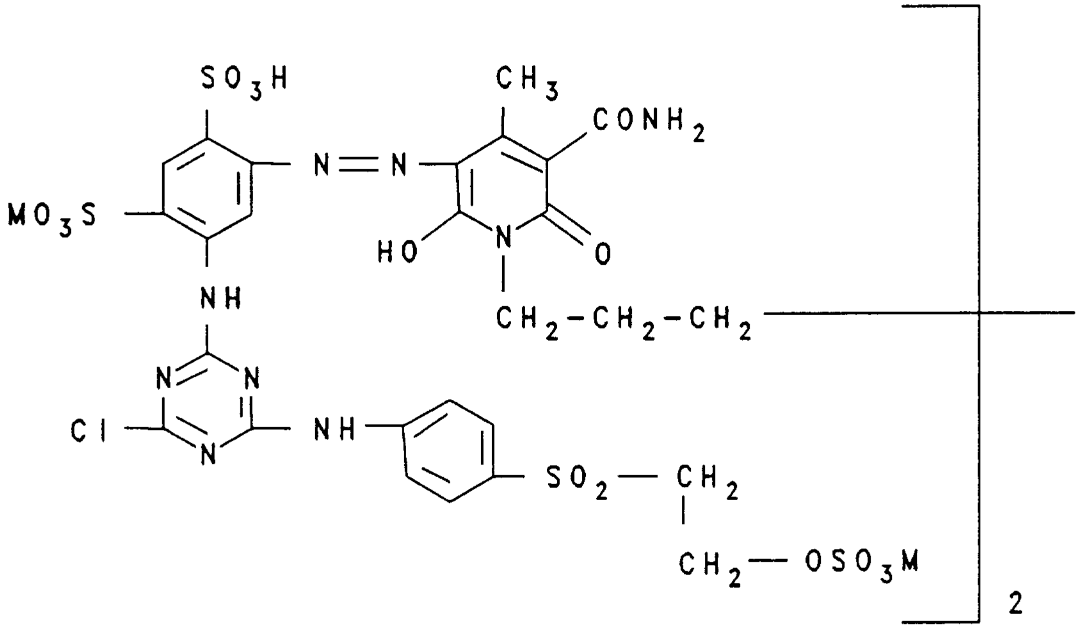

in welcher M Wasserstoff oder ein Alkalimetall ist.

**8.** Verfahren zur Herstellung einer Disazoverbindung der allgemeinen Formel (1) von Anspruch 1, dadurch gekennzeichnet, daß man eine Diazoniumverbindung eines Amins der allgemeinen Formel D-$NH_2$ mit D der in Anspruch 1 genannten Bedeutung oder die Diazoniumverbindungen zweier Amine der allgemeinen Formel D-$NH_2$ in der zweifach äquivalenten Menge mit einer Verbindung der allgemeinen Formel (4)

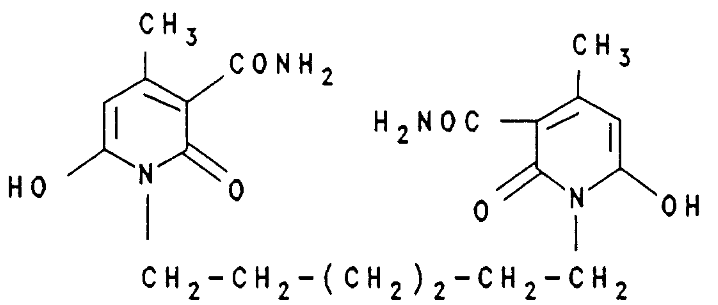

(4)

bei einer Temperatur zwischen 0 und 50 ° C und einem pH-Wert zwischen 3 und 7 umsetzt.

**9.** Verwendung einer Verbindung von Anspruch 1 zum Färben (einschließlich Bedrucken) von hydroxy- und/oder carbonamidgruppenhaltigem Material, insbesondere Fasermaterial.

**10.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt und den Farbstoff auf dem Material mittels Wärme oder mit Hilfe eines alkalisch wirkenden Mittels oder mittels beider Maßnahmen fixiert, dadurch gekennzeichnet, daß man als Farbstoff ein Verbindung von Anspruch 1 einsetzt.

**11.** Verbindung der Formel (4)

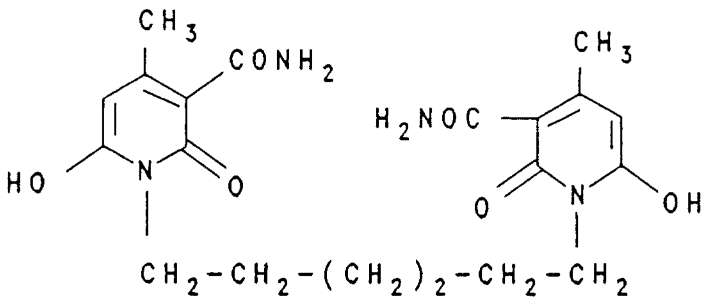

(4)

**12.** Verfahren zur Herstellung einer Verbindung der Formel (4) von Anspruch 11, dadurch gekennzeichnet, daß man N,N'-Di-cyanoacetyl-1,6-diamino-hexan mit Acetessigsäureethylester umsetzt und in der erhaltenen Verbindung die beiden Cyanogruppen zu Carbamoylgruppen hydrolysiert.

**13.** Verwendung einer Verbindung der Formel (4) von Anspruch 11 zur Synthese von Azofarbstoffen, insbesondere von Azofarbstoffen der allgemeinen Formel (1) von Anspruch 1.

Europäisches Patentamt

# EUROPÄISCHER RECHERCHENBERICHT

Nummer der Anmeldung
EP 95 10 5626

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X<br><br><br><br><br><br><br>D | CHEMICAL ABSTRACTS, vol. 98, no. 18, 2.Mai 1983<br>Columbus, Ohio, US;<br>abstract no. 145029z, 'Fiber-reactive disazo dyes'<br>Seite 87;<br>* Zusammenfassung *<br>& JP-A-57 161 175 (SUMITOMO CHEMICAL CO., LTD.) 4.Oktober 1982<br>* Tabellen; Formeln *<br>--- | 1-13 | C09B62/513<br>C09B62/09<br>C07D213/82 |
| X<br><br><br><br><br><br><br>D | CHEMICAL ABSTRACTS, vol. 100, no. 12, 19.März 1984<br>Columbus, Ohio, US;<br>abstract no. 87246q, 'Reactive disazo dyes for cotton'<br>Seite 78;<br>* Zusammenfassung *<br>& JP-A-58 168 660 (SUMITOMO CHEMICAL CO., LTD.) 5.Oktober 1983<br>* Tabellen; Formeln *<br>----- | 1-13 | |
| | | | **RECHERCHIERTE SACHGEBIETE (Int.Cl.6)** |
| | | | C09B<br>C07D |

Der vorliegende Recherchenbericht wurde für alle Patentansprüche erstellt

| Recherchenort | Abschlußdatum der Recherche | Prüfer |
|---|---|---|
| DEN HAAG | 7.August 1995 | Ginoux, C |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument